(19) Europäisches Patentamt
European Patent Office
Office européen des brevets

(11) EP 0 728 790 B1

(12) EUROPÄISCHE PATENTSCHRIFT

(45) Veröffentlichungstag und Bekanntmachung des Hinweises auf die Patenterteilung:
**03.05.2000 Patentblatt 2000/18**

(51) Int Cl.[7]: **C08G 65/18**, C08G 65/22, A61K 6/087, C08L 71/02

(21) Anmeldenummer: **96101788.6**

(22) Anmeldetag: **08.02.1996**

(54) **Polymerisierbares Material**

Polymerisable material

Matériau polymérisable

(84) Benannte Vertragsstaaten:
**CH DE FR GB IT LI**

(30) Priorität: **22.02.1995 DE 19506222**

(43) Veröffentlichungstag der Anmeldung:
**28.08.1996 Patentblatt 1996/35**

(73) Patentinhaber: **Heraeus Kulzer GmbH & Co.KG
63450 Hanau (DE)**

(72) Erfinder:
- **Schaefer, Roland, Dr.
D-61381 Friedrichsdorf (DE)**
- **Heindl, Detlef, Dr.
D-35796 Weinbach (DE)**
- **Schödel, Dieter, Dr.
D-65193 Wiesbaden (DE)**
- **Nuyken, Oskar, Prof. Dr.
D-81927 München (DE)**
- **Böhner, Ralf, Dr.
D-82151 Martinsried (DE)**
- **Erdmann, Christoph
D-85221 Dachau (DE)**

(74) Vertreter: **Kühn, Hans-Christian
Heraeus Holding GmbH,
Stabsstelle Schutzrechte,
Heraeusstrasse 12-14
63450 Hanau (DE)**

(56) Entgegenhaltungen:
**EP-A- 0 345 073** **EP-A- 0 438 629**
**DE-B- 1 023 227** **GB-A- 1 048 949**

- **MACROMOLECULES, Bd. 28, Nr. 16, 16.Januar 1992, Seiten 651-652, XP002004449 TAKEUCHI D. ET AL: "Lewis acid-promoted anionic polymerisation of a monomer with high cationic polymerisability "**
- **DIE ANGEWANDTE MAKROMOLEKULARE CHEMIE , Bd. 135, Nr. 2173, 1985, Seiten 131-138, XP002004450 KLEMM E.: "Versuche zur stufenweise epoxid/methacrylate-photopolymerisation mit diaryliodoniumsalz-photoinitiatoren"**
- **J. MACROMOLECULAR SCIENCE , Bd. A29, Nr. 10, 1992, Seiten 915-930, XP002005295 SASAKI H. ET AL : "The synthesis ,characterisation and photoinitiated cationic polymerisation of difunctional oxetanes"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein polymerisierbares Material, das mindestens einen Polymerisationsinitiator enthält.

Die Erfindung betrifft besonders ein polymerisierbares Material für medizinische und dentale Zwecke, zum Beispiel für den Einsatz als Knochenzement und zur Herstellung von Zahnfüllungen, Kronen, Brücken, Verblendungen, Inlays, künstlichen Zähnen und weichen Zahnprothesen-Unterfütterungen, und zur Herstellung von Beschichtungen.

Polymerisierbare Dentalmaterialien sind seit vielen Jahren bekannt. Die ersten dieser Materialien bestanden aus Mischungen von monomerem mit polymerem Methylmethacrylat, die nach Zusatz eines Katalysators bzw. eines Systems aus Katalysator und Beschleuniger bei Raumtemperatur (Kaltpolymerisation) oder bei erhöhter Temperatur (Heißpolymerisation) aushärteten.

Eine Verbesserung mechanischer Eigenschaften der ausgehärteten Produkte wurde durch Dentalmaterialien, die zusätzlich feinteilige Füllstoffe, wie Quarz oder Aluminiumsilicate, enthalten, eine Verbesserung der ästhetischen Wirkung durch die Entwicklung neuer Katalysator-Systeme, die keine Verfärbung mehr verursachen, und eine Verringerung der Polymerisationsschrumpfung durch die Verwendung von Methacrylsäureestern höherer Alkohole neben oder anstelle von Methylmethacrylat erreicht.

Auf dem Gebiet der durch Polymerisation aushärtenden Zahnfüllungsmaterialien bedeutete es einen großen Fortschritt, als Rafael L. Bowen anstelle des bis dahin darin verwendeten Methylmethacrylats langkettige monomere Dimethacrylate - Reaktionsprodukte des Bisphenol A und seiner Derivate mit Glycidylmethacrylat, besonders das sogenannte Bis-GMA, - und zur Verstärkung der Kunststoff-Matrix feines Quarzglas-Pulver einführte (US 3 066 112 A).

Ein Beispiel für ein weiteres Komposit - ein Dentalmaterial, das neben organischen Monomeren einen feinteiligen anorganischen Füllstoff enthält - wird in US 3 539 533 A beschrieben. Das polymerisierbare Bindemittel ist dabei ein Gemisch aus Bis-GMA, Bisphenol A-dimethacrylat, verdünnendem Monomer, besonders Triethylenglykoldimethacrylat, und gegebenenfalls Methacrylsäure in geringer Menge, das zusammen mit etwa 65 - 75 Gewichts-% des anorganischen Füllstoffs, zum Beispiel aus Siliciumdioxid, Glas, Aluminiumoxid oder Quarz, verwendet wird. Der anorganische Füllstoff kann eine Teilchengröße von etwa 2 - 85 Mikrometer besitzen; zur Verbesserung des Verbundes Kunststoff/Füllstoff wird er mit einem Silan, wie zum Beispiel 3-Methacryloyloxypropyltrimethoxysilan, vorbehandelt.

Der Einsatz mikrofeiner anorganischer Füllstoffe mit mittleren Teilchengrößen von 0,01 - 0,4 Mikrometer führte dann zu auch in ästhetischer Hinsicht verbesserten dentalen Kunststoff-Produkten mit Hochglanzpolierbarkeit und einer Transparenz, die der natürlicher Zähne ähnlich ist (DE 24 03 211 C3).

Einen weiteren Schritt in der Entwicklung von Dentalmaterialien auf Kunststoff-Basis stellen die sogenannten Hybrid-Materialien dar, die sowohl mikrofeine Füllstoffe als auch konventionelle Füllstoffe (Makrofüllstoffe) enthalten. Ein solches Dentalmaterial ist zum Beispiel aus DE 24 05 578 C3 bekannt. Es enthält 30 - 80 Gewichts-% einer Mischung aus durch Flammhydrolyse hergestellter amorpher Kieselsäure (pyrogenes Siliciumdioxid) mit einer maximalen Teilchengröße von 0,07 Mikrometer und feinteiligem Glas, bevorzugt Borsilicatglas, Barium- oder Lanthanoxid enthaltendes Glas oder Lithiumaluminiumsilicatglas, mit einer Teilchengröße bis zu 5 Mikrometer.

Ebenfalls ein Hybrid-Material stellt die in EP 382 033 A2 beschriebene Dentalmasse dar, die neben polymerisierbaren Acrylaten oder Methacrylaten und einem Katalysator für die Photopolymerisation (Photoaktivator) 5 - 80 Gewichts-% eines silanisierten Glases oder einer silanisierten Glaskeramik mit einer mittleren Teilchengröße zwischen 0,1 und 10 Mikrometer und 2 - 10 Gewichts-% eines oberflächenbehandelten Mikrofüllers enthält.

Eines der Ziele bei der Entwicklung neuer Dentalmaterialien ist es, die Schrumpfung während der Polymerisation zu verringern.

In EP 0 235 826 B1 werden als Monomere für schrumpfungsarm polymerisierbare Dentalmassen im Alkohol-Rest (Meth)Acryloyloxy-Gruppen aufweisende Triglykolsäurediester vorgeschlagen. Mit diesen Monomeren lassen sich Dentalmassen herstellen, deren Schrumpfungseigenschaften bei geringerem Füllstoff-Gehalt denen von hochgefüllten Massen entsprechen.

Aus DE 38 19 777 A1 sind polymerisierbare Massen bekannt, die 15 - 60 Gewichts-% Monomere mit radikalisch polymerisierbaren Doppelbindungen, vorzugsweise Acryl- und Methacrylsäureester, 8 - 40 Gewichts-% faserförmige, anorganische Füllstoffe, 15 - 60 Gewichts-% nichtfaserförmige, anorganische Füllstoffe und 0 - 10 Gewichts-% Hilfsmittel enthalten. Die Massen lassen sich durch Polymerisation (Raumtemperatur, erhöhte Temperatur, Einwirkung von Licht) zu Formkörpern aushärten, die für technische, medizinische und zahnmedizinische Anwendungen geeignet sind. Während der Härtung tritt nur eine geringe Polymerisationsschrumpfung auf.

Nach DE 40 01 977 C2 und DE 40 01 978 C2 lassen sich Mischungen mit geringer Polymerisationsschrumpfung aus monomeren Estern der Acrylsäure und/oder Methacrylsäure oder aus Vinylethern und darin löslichen Polymeren herstellen. Die Polymerisation der besonders für dentale Zwecke geeigneten Mischungen kann durch Kaltpolymerisation, Heißpolymerisation und Photopolymerisation erfolgen.

In DE 42 29 947 A1 werden schrumpfungsarm polymerisierbare Dentalzemente und Knochenzemente beschrieben, die gegenüber den bisher verwendeten Zementen verbesserte mechanische Eigenschaften aufweisen. Die Zemente enthalten Oligomere und/oder Polymere von Vinyldicarbonsäureanhydriden und/oder deren Copolymere mit

anderen Vinylverbindungen, mit diesen unter Ringöffnung reagierende mono- und/oder oligo- und/oder polyhydroxyfunktionelle Verbindungen, reaktive anorganische Füllstoffe, welche mit den bei der Ringöffnung freiwerdenden Carboxylgruppen reagieren können, sowie Härtungsmittel.

Die Aufgabe der Erfindung ist es, ein schrumpfungsarm polymerisierbares Material zu finden, das besonders für medizinische und dentale Zwecke und zur Herstellung von Beschichtungen geeignet ist.

Das die Lösung der Aufgabe darstellende Material ist erfindungsgemäß dadurch gekennzeichnet, daß es eine Mischung aus 50 - 90 Gewichts-% einer oder mehrerer kationisch polymerisierbarer Verbindungen der unten dargestellten Formeln und 10 - 50 Gewichts-% eines oder mehrerer radikalisch polymerisierbarer Methacrylsäureester enthält, sowie 0,3-4 Gew % eines kationischen Polymerisationsinitiator und 0,1-5 Gew % eines Campherchino/Amin-Systems als radikalischen Polymerisationsinitiator. Die angegebene Menge an kationischem Polymerisationsinitiator bezieht sich auf die Gesamtmenge aus kationisch polymerisierbaren Verbindungen und kationischem Polymerisationsinitiator. Entsprechend bezieht sich die angegebene Menge an radikalischem Polymerisationsinitiator auf die Gesamtmenge aus radikalisch polymerisierbaren Methacrylsäureestern und radikalischem Polymerisationsinitiator.

Die kationisch polymerisierbaren Verbindungen weisen die allgemeinen Formeln aus:

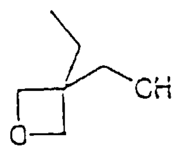

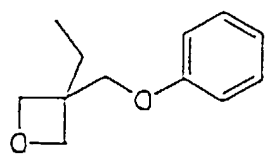

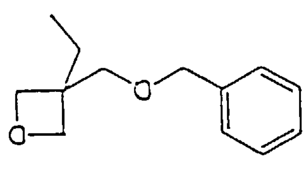

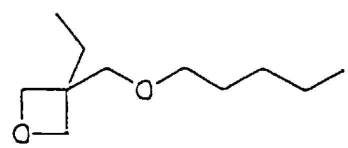

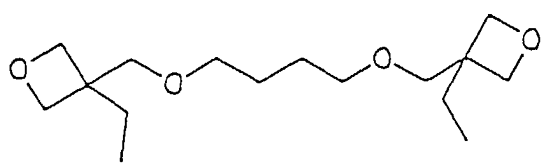

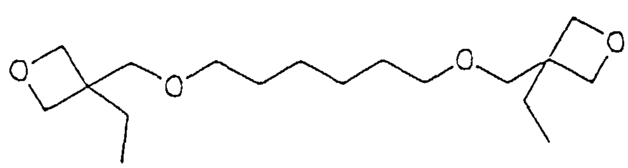

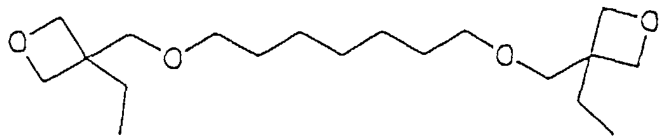

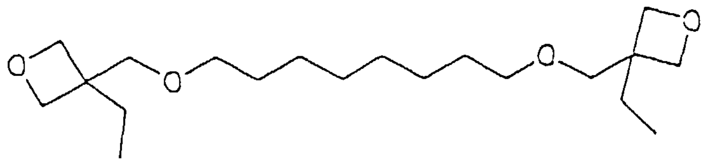

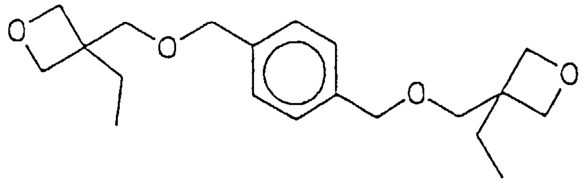

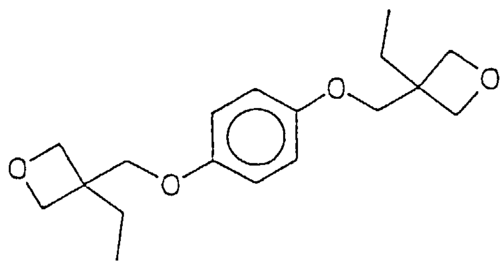

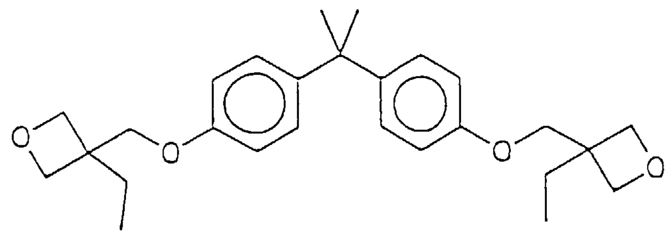

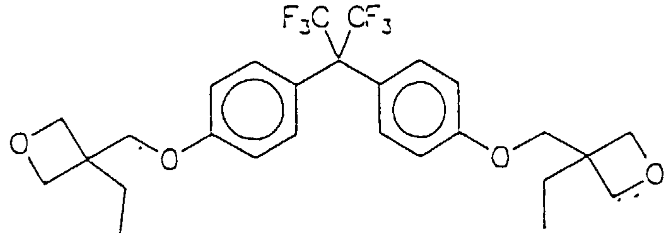

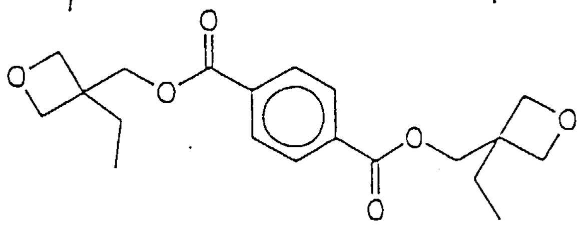

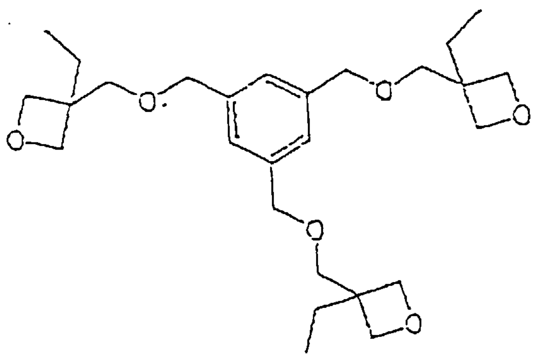

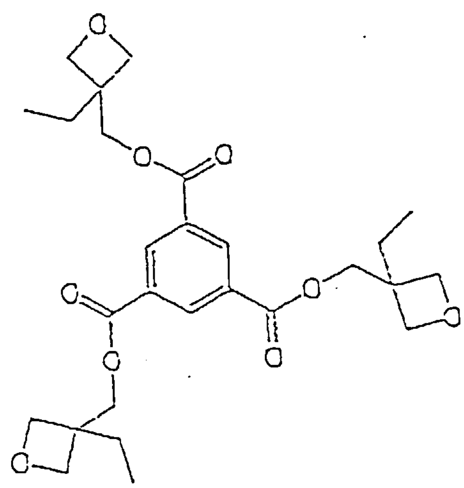

Die kationisch polymerisierbaren Verbindungen gehören zu der an sich bekannten Gruppe der Oxetan- oder Oxacyclobutan-Derivate, wie sie zum Beispiel in J. Am. Chem. Soc. 79 (1957), 3455 und 3456, und J. Macromol. Sci. A29(10), 915 - 930 (1992), und A30(2&3), 189 - 206 (1993) beschrieben werden. Ihre Darstellung kann entsprechend der dort angegebenen Weise erfolgen.

Überraschenderweise unterliegt das erfindungsgemäße Material einer nur geringen Schrumpfung während der Polymerisation. Ein weiterer Vorteil ist die - verglichen mit Methacrylsäureestern - relative Unempfindlichkeit der Polymerisationsreaktion gegenüber Sauerstoff.

Die Polymerisation der kationisch polymerisierbaren Verbindungen wird durch Säuren, auch Lewissäuren, initiiert. Beispiele dafür sind Trifluoressigsäure, Schwefelsäure und Bortrifluorid. Die Photopolymerisation kann in Gegenwart von Oniumverbindungen als kationische Polymerisationsinitiatoren erfolgen. Besonders bewährt haben sich dabei Sulfoniumsalze, lodoniumsalze, Isochinoliniumsalze und Cyclopentadienyleisen(I)-salze. Bevorzugt werden [4-Diphenylsulfonio)-phenyl]-phenylsulfid-hexafluorphosphat, Bis-[4-diphenylsulfonio)phenyl]-sulfid-bis-hexafluorphosphat und (4-Pentadecyloxyphenyl)-phenyliodonium-hexafluorphosphat und die entsprechenden Hexafluorarsenate, Hexafluorantimonate und Tetrafluorborate. Durch Sensibilisatoren, wie Anthracen und Perylen, kann die spektrale Empfindlichkeit des Initiators erhöht werden.

Für ein radikalisch polymerisierbare Methacrylsäureester enthaltendes Material haben sich Ester der Methacrylsäure mit Mono-, Di- und Polyolen bewährt. Dafür geeignete Polymerisationsinitiatoren sind Campherchinon/Amin-Systeme (GB 1 408 265 B).

In den Materialien können auch an sich bekannte feinteilige organische Füllstoffe, anorganische Füllstoffe oder Gemische daraus enthalten sein. Beispiele für organische Füllstoffe sind die Homo- und Copolymerisate von Alkylmethacrylaten, zum Beispiel Methylmethacrylat. Anorganische Füllstoffe können aus Glas, Keramik oder Glaskeramik bestehen. Beispiele dafür sind Lithium-, Barium-, Strontiumaluminiumsilicatglas, Bariumaluminiumborosilicatglas, Quarz und Siliciumdioxid, letzteres besonders in Form sehr feiner Teilchen (durch Fällung oder Flammhydrolyse gewonnenes Silicumdioxid). Die anorganischen Füllstoffe können mit einem Silan, zum Beispiel 3-Methacryloyloxypropyltrimethoxysilan, behandelt sein.

Sollen sich die Materialien für medizinische und dentale Zwecke eignen, so müssen die sie bildenden Bestandteile so ausgewählt werden, daß die an solche Materialien gestellten physikalischen, chemischen, anwendungstechnischen und ästhetischen Anforderungen erfüllt werden. Die Formulierung solcher Materialien ist dem Fachmann bekannt.

Es hat sich gezeigt, daß das erfindungsgemäße Material auch sehr gut zur Herstellung von Beschichtungen auf zum Beispiel Metall, Keramik, Glas, Holz, Papier und Kunststoff benutzt werden kann.

Zur näheren Erläuterung der Erfindung werden in den folgenden Beispielen die Darstellung einiger kationisch polymerisierbarer Verbindungen, nämlich von 3-Ethyl-3-hydroxymethyloxetan (Oxetan I), 3-Ethyl-3-hydroxymethyloxetanbenzylether (Oxetan II) und 3,3'-(p-Xylylendioxymethyl)-bis-(3-ethyloxetan) (Oxetan III), die Bestimmung der Schrumpfung während der Polymerisation dieser drei Oxetane und - zum Vergleich - von bekannten Methacrylsäureestern (Beispiel 4) und zwei polymerisierbare Materialien gemäß der Erfindung und ihre Polymerisation (Beispiele 5 und 6) beschrieben.

Beispiel 1

Darstellung von 3-Ethyl-3-hydroxymethyloxetan (Oxetan I)

In einem Kolben werden unter Schutzgas 59 g Diethylcarbonat (0,5 mol), 67 g Trimethylpropan (0,5 mol) und 50 mg Kaliumhydroxid, gelöst in 2 ml absolutem Ethanol, bei Raumtemperatur gemischt. Der Reaktionsansatz wird 1 Stunde bei 105 - 110° C Badtemperatur unter Rückfluß erhitzt. Danach wird der Rückflußkühler durch eine Destillationsbrücke ersetzt und der Ethanol bei einer Badtemperatur von 120° C abdestilliert. Danach wird das 3-Ethyl-3-hydroxymethyloxetan langsam unter Vakuum und vorsichtigem Erhöhen der Badtemperatur auf 180° C abdestilliert. Die Erhöhung der Temperatur muß langsam erfolgen, da Kohlendioxid freigesetzt wird. Die erhaltene farblose Flüssigkeit wird anschließend über eine Kolonne fraktionierend destilliert.

| | |
|---|---|
| Ausbeute | 45,3 g (0,39 mol); 78 % der Theorie |
| Siedepunkt | 117° C (20 mbar) |
| ρ | 1,0209 g $cm^{-3}$ |

Beispiel 2

Darstellung von 3-Ethyl-3-hydroxymethyloxetanbenzylether (Oxetan II)

In einem Kolben werden 42,8 g (0,25 mol) Benzylbromid und 23,2 (0,20 mol) 3-Ethyl-3-hydroxymethyloxetan vorgelegt. Dazu werden nacheinander bei 0° C 100 g einer 50%igen wässerigen Kaliumhydroxid-Lösung und 2,0 g Tetra-n-butylammoniumbromid gegeben. Die entstandene Suspension wird anschließend 24 Stunden lang bei Raumtemperatur gerührt. Der Reaktionsansatz wird in einen Scheidetrichter gegeben und einmal mit Diethylether ausgeschüttelt. Die Diethylether-Phase wird zweimal mit Wasser gewaschen und über Natriumsulfat getrocknet. Dann wird der Diethylether abgezogen und der Rückstand unter Vakuum fraktionierend destilliert.

| | |
|---|---|
| Ausbeute | 24,1 g (0,117 mol); 58 % der Theorie |
| Siedepunkt | 82° C (0,02 mbar) |
| ρ | 1,0263 g $cm^{-3}$ |

Beispiel 3

Darstellung von 3,3'-(p-Xylylendioxymethyl)-bis-(3-ethyloxetan) (Oxetan III)

In einem Kolben werden 8,7 g (0,033 mol) 1,4-Dibromxylol (1,4-Dibrommethylbenzol) und 11,6 g (0,100 mol) 3-Ethyl-3-hydroxymethyloxetan vorgelegt. Dazu werden nacheinander bei 0° C 17 g einer 50%igen wässerigen Kaliumhydroxid-Lösung und 0,33 g Tetra-n-butylammoniumbromid gegeben. Die entstandene Suspension wird anschließend 24 Stunden lang bei Raumtemperatur gerührt. Der Reaktionsansatz wird in einen Scheidetrichter gegeben und einmal mit Diethylether ausgeschüttelt. Die Diethylether-Phase wird zweimal mit Wasser gewaschen und über Natriumsulfat getrocknet. Dann wird der Diethylether abgezogen und der Rückstand unter Vakuum fraktionierend destilliert.

| | |
|---|---|
| Ausbeute | 8,4 g (0,025 mol); 76 % der Theorie |
| Siedepunkt | 173° C (0,01 mbar) |
| Schmelzpunkt | 32° C |
| ρ | 1,0652 g $cm^{-3}$ |

Beispiel 4

Bestimmung der Schrumpfung während der Polymerisation

Die Polymerisation von 3-Ethyl-3-hydroxymethyloxetan (Oxetan I), 3-Ethyl-3-hydroxymethyloxetanbenzylether (Oxetan II) und 3,3'-(p-Xylylendioxymethyl)-bis-(3-ethyloxetan) (Oxetan III) erfolgte im Belichtungsgerät Unilux AC der Heraeus Kulzer GmbH. Dabei wurde wie folgt vorgegangen: Zwischen zwei Quarzglasplatten wurde mit herkömmlichem Haushaltskleber ein Teflonring von einem Millimeter Stärke geklebt. In diesen Ring wurde dann das Monomer, das zusammen mit dem Photoinitiator (Degacure KI 85[1]) in dunklen Flaschen aufbewahrt wurde, mittels einer Spritze aufgebracht. Wichtig dabei war, daß bei diesem Schritt keine Luftblasen in den Ring eingebracht wurden. Die so präparierten Quarzglasscheiben wurden im Abstandshalter eines IR-Gerätes fixiert und waagrecht auf den Tisch des Belichtungsgerätes gestellt. Dann erfolgte die Polymerisation mit 20 Bestrahlungseinheiten.

Die Dichte der Monomeren wurde bei 20° C mit einem digitalen Dichtemeßgerät gemessen. Die Bestimmung wird dabei auf elektronische Frequenz- beziehungsweise Schwingungsdauermessung zurückgeführt.

Die Dichte der Polymeren wurde nach der Schwebemethode bestimmt. Diese Messung wurde vier Wochen nach der eigentlichen Polymerisation durchgeführt, um die Nachpolymerisation möglichst abzuschließen. Um reproduzierbare Ergebnisse zu erhalten, wurden jeweils drei Polymerstücke unterschiedlicher Größe einer Charge verwendet. Dabei wurde beobachtet, ob diese in einer Salzlösung zu Boden sinken oder aufsteigen. Mit einem Aerometer wurde dann die Dichte der Salzlösung bestimmt. Dieser Schritt wurde dreimal wiederholt und der Mittelwert berechnet.

Die Schrumpfung wird nach folgender Formel berechnet:

$$\text{Schrumpfung} = \frac{\text{Dichte(Monomer) - Dichte(Polymer)}}{\text{Dichte(Monomer)}} * 100\%$$

Die so bestimte Polymerisationsschrumpfung S der drei Oxetane und von zwei Methacrylsäureestern, das

Molekulargewicht und das Verhältnis von Schrumpfung zu Molekulargewicht S/MG werden in der Tabelle angegeben.

| Monomer | Schrumpfung S [Vol-%] | Molekulargewicht MG | S/MG |
|---|---|---|---|
| Oxetan I | 7,8 | 116 | 0,067 |
| Oxetan II | 4,9 | 216 | 0,022 |
| Oxetan III | 3,9 | 334 | 0,011 |
| Methylmethacrylat | 21,3 | 100 | 0,213 |
| Triethylenglykoldimethacrylat | 12,0 | 286 | 0,044 |

Beispiel 5

Durch Photopolymerisation aushärtbares Material

| | Gewichts-% |
|---|---|
| Oxetan III | 39,0 |
| Oxetan I | 10,0 |
| Bis-GMA | 48,0 |
| Degacure KI 85[1] | 2,5 |
| Lucirin LR8728[2] | 0,5 |

[1] Bis-[4-(diphenylsulfonio)-phenyl]-sulfid-bis-hexafluorphosphat
[2] 2,4,6-Trimethylbenzoyldiphenylphosphinoxid

Die Aushärtung erfolgt durch Bestrahlung in dem Lichtgerät Dentacolor XS (Heraeus Kulzer GmbH) innerhalb von 90 Sekunden.

Beispiel 6

Durch Photopolymerisation aushärtbares Material

| | Gewichts-% |
|---|---|
| Oxetan III | 9,75 |
| Oxetan I | 2,50 |
| Bis-GMA | 12,00 |
| Degacure KI 85[1] | 0,62 |
| Lucirin LR 8728[2] | 0,13 |
| Lithiumaluminiumsilicat, mittlere Teilchengröße 5 Mikrometer | 75,00 |

[1] Bis-[4-(diphenylsulfonio)-phenyl]-sulfid-bis-hexafluorphosphat
[2] 2,4,6-Trimethylbenzoyldiphenylphosphinoxid

Die Aushärtung erfolgt durch Bestrahlung in dem Lichtgerät Translux (Heraeus Kulzer GmbH) innerhalb von 60 Sekunden.

**Patentansprüche**

1. Polymerisierbares Material, das mindestens einen Polymerisationsinitiator enthält, dadurch gekennzeichnet, daß es eine Mischung aus 50 - 90 Gewichts-% einer oder mehrerer kationisch polymerisierbarer Verbindungen und eines kationischen Polymerisationsinitiators dafür und 10 - 50 Gewichts-% eines oder mehrerer radikalisch polymerisierbarer Methacrylsäureester und eines Campherchinon/Amin-Systems als radikalischen Polymerisationsinitiator dafür enthält, wobei der Gehalt an kationischem Polymerisationsinitiator 0,3 - 4 Gewichts-% und der an radikalischem Polymerisationsinitiator 0,1 - 5 Gewichts-% beträgt und die kationisch polymerisierbaren Verbindungen den folgenden Formeln entsprechen.

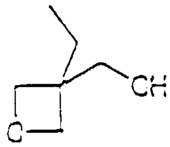

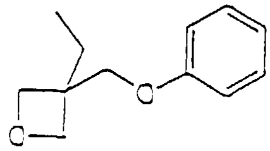

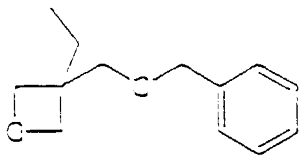

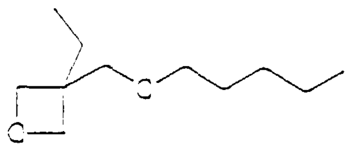

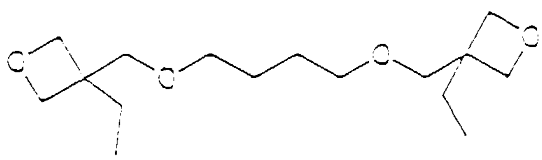

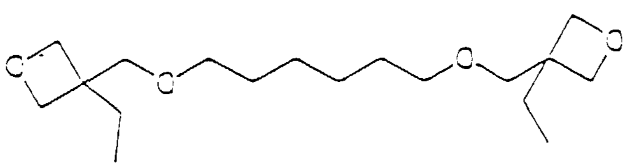

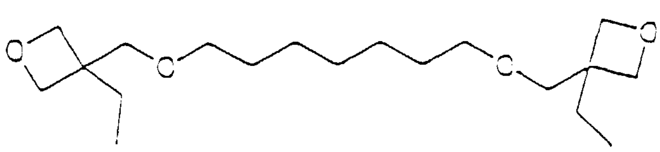

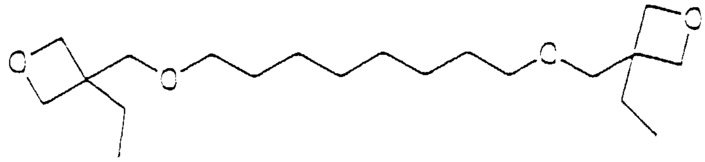

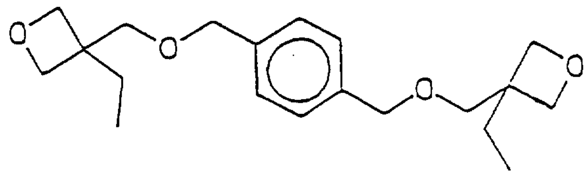

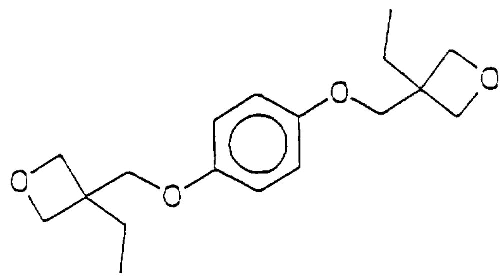

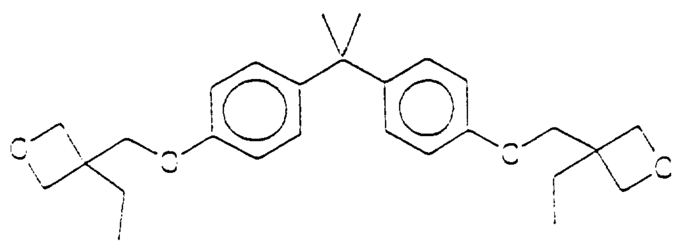

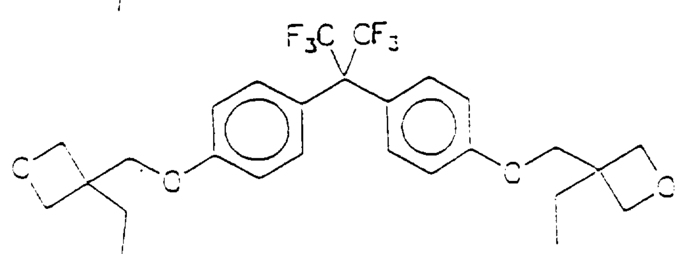

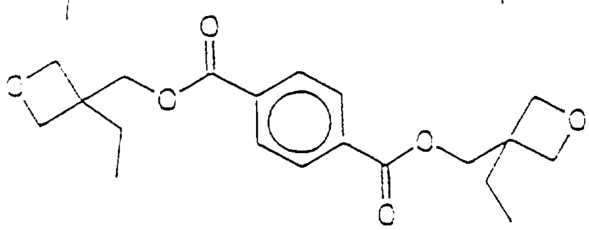

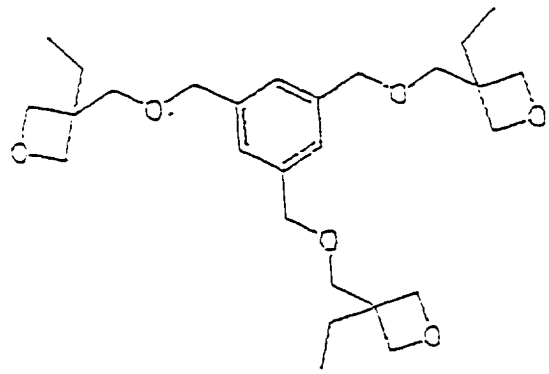

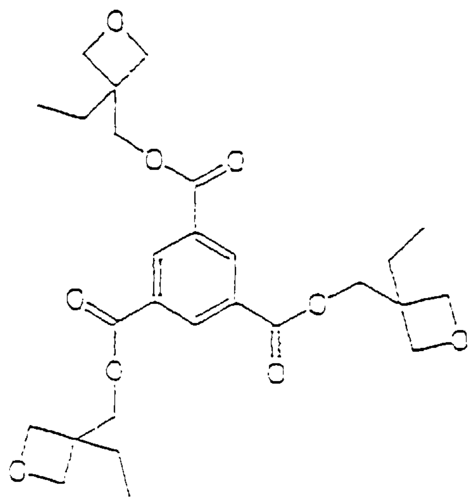

2. Polymerisierbares Material nach Anspruch 1, dadurch gekennzeichnet, daß der Gehalt an kationischem Polymerisationsinitiator 0,5 - 1,5 Gewichts-% und der Gehalt an radikalischem Polymerisationsinitiator 0,3 - 1 Gewichts-% beträgt.

3. Polymerisierbares Material nach Anspruch 1 oder 2, dadurch gekennzeichnet, daß der kationische Polymerisationsinitiator ein Sulfoniumsalz für die Photopolymerisation ist.

4. Polymerisierbares Material nach Anspruch 1 oder 2, dadurch gekennzeichnet, daß der kationische Polymerisationsinitiator ein Iodoniumsalz für die Photopolymerisation ist.

5. Polymerisierbares Material nach einem der Ansprüche 1 bis 4, dadurch gekennzeichnet, daß es 20 - 95 Gewichts-% der Mischung und 5 - 80 Gewichts-% feinteiligen organischen Füllstoff, feinteiligen anorganischen Füllstoff oder ein Gemisch daraus enthält.

6. Polymerisierbares Material nach Anspruch 5, dadurch gekennzeichnet, daß der feinteilige organische Füllstoff aus Alkylmethacrylat-Homo- oder -Copolymerisaten besteht.

7. Polymerisierbares Material nach Anspruch 5 oder 6, dadurch gekennzeichnet, daß der feinteilige anorganische Füllstoff aus Lithiumaluminiumsilicatglas, Bariumaluminiumsilicatglas, Strontiumaluminiumsilicatglas, Bariumaluminiumborosilicatglas, Siliciumdioxid oder einem Gemisch daraus besteht.

**8.** Verwendung des polymerisierbaren Materials nach einem der Ansprüche 1 bis 7 als Dentalmaterial.

**9.** Verwendung des polymerisierbaren Materials nach einem der Ansprüche 1 bis 7 als Knochenzement.

**10.** Verwendung des polymerisierbaren Materials nach einem der Ansprüche 1 bis 7 zur Herstellung von Beschichtungen auf Keramik, Glas, Metall, Holz, Papier und Kunststoff.

## Claims

**1.** Polymerizable material which contains at least one polymerization initiator, characterized in that it contains a mixture of 50 - 90% by weight of one or more cationically polymerizable compounds and of a cationic polymerization initiator therefor and 10 - 50 % by weight of one or more methacrylic esters capable of free radical polymerization and of a camphorquinone/amine system as a free radical polymerization initiator for said ester or esters, the content of cationic polymerization initiator being 0.3 - 4% by weight and that of free radical polymerization initiator being 6.1 - 5% by weight and the cationically polymerizable compounds corresponding to the following formulae.

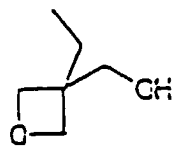

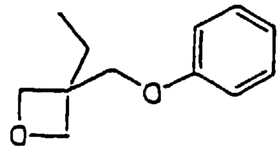

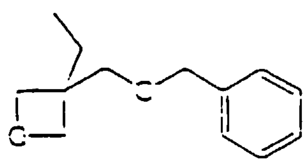

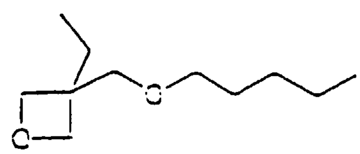

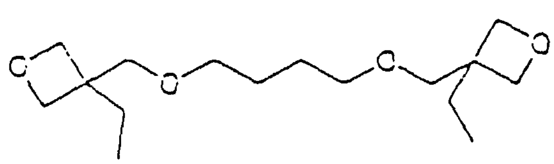

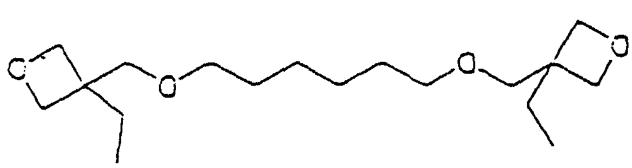

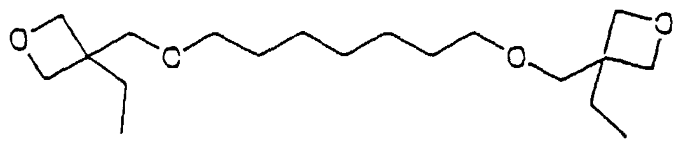

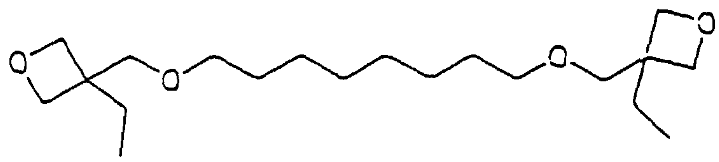

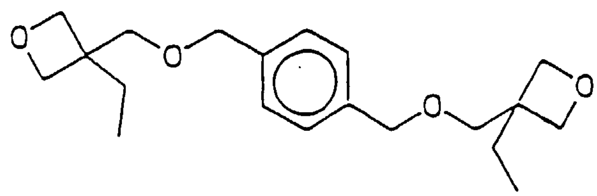

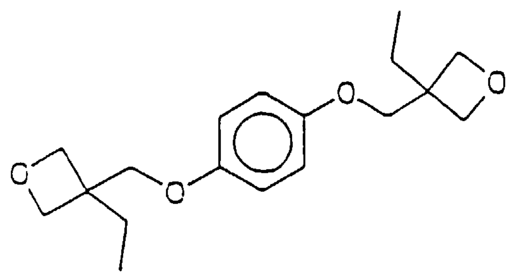

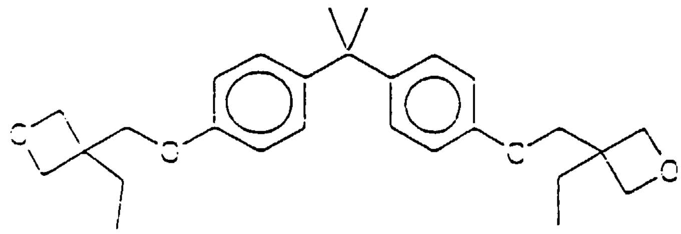

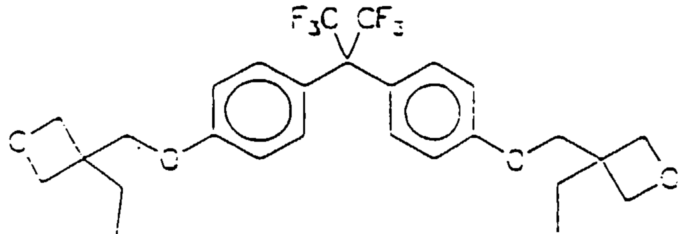

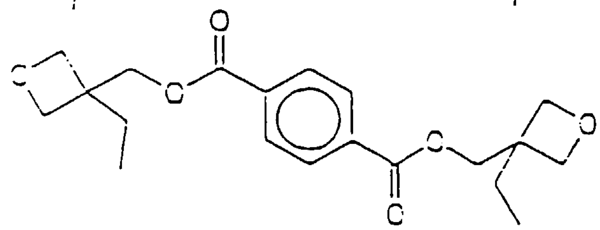

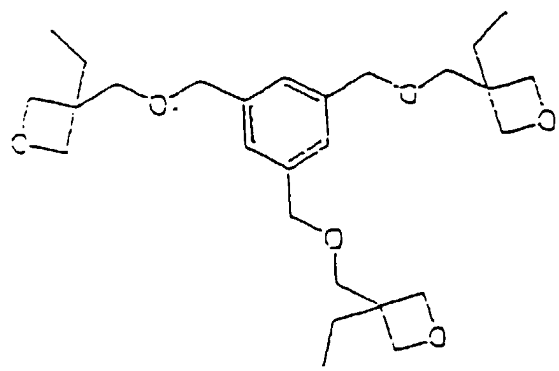

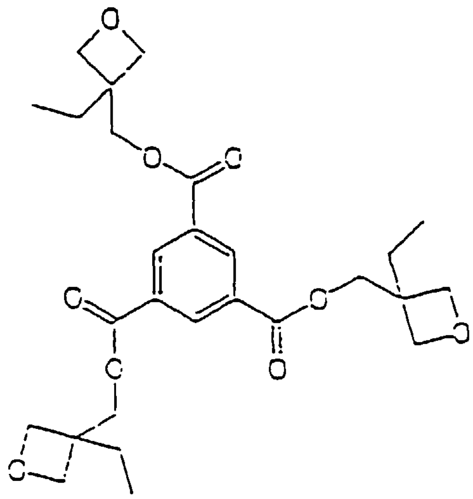

2. Polymerizable material according to Claim 1, characterized in that the content of cationic polymerization initiator is 0.5 - 1.5% by weight and the content of free radical polymerization initiator is 0.3 - 1% by weight.

3. Polymerizable material according to Claim 1 or 2, characterized in that the cationic polymerization initiator is a sulphonium salt for the photopolymerization.

4. Polymerizable material according to Claim 1 or 2, characterized in that the cationic polymerization initiator is an iodonium salt for the photopolymerization.

5. Polymerizable material according to any of Claims 1 to 4, characterized in that it contains 20 - 95% by weight of the mixture and 5 - 80% by weight of finely divided organic filler, finely divided inorganic filler or a mixture thereof.

6. Polymerizable material according to Claim 5, characterized in that the finely divided organic filler consists of alkyl methacrylate homo- or copolymers.

7. Polymerizable material according to Claim 5 or 6, characterized in that the finely divided inorganic filler consists of lithium aluminium silicate glass, barium aluminium silicate glass, strontium aluminium silicate glass, barium aluminium borosilicate glass, silicon dioxide or a mixture thereof.

8. Use of the polymerizable material according to any of Claims 1 to 7 as dental material.

9. Use of the polymerizable material according to any of Claims 1 to 7 as bone cement.

10. Use of the polymerizable material as claimed in any of Claims 1 to 7 for the production of coatings on ceramic, glass, metal, wood, paper and plastics.

**Revendications**

1. Matériau polymérisable qui contient au moins un initiateur de polymérisation, caractérisé en ce qu'il contient un mélange de 50-90% en poids d'un ou plusieurs composés cationiquement polymérisables et d'un initiateur de polymérisation cationique pour ceux-ci et de 10-50% en poids d'un ou plusieurs esters d'acide méthacrylique radicalairement polymérisables et d'un système de camphroquinone/amine en tant qu'initiateur de polymérisation radicalaire, la teneur en initiateur de polymérisation cationique étant de 0,3-4% en poids et celle en initiateur de polymérisation radicalaire de 0,1-5% en poids et les composés cationiquement polymérisables correspondant aux formules suivantes:

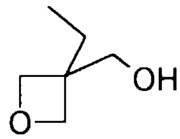
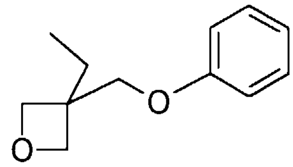

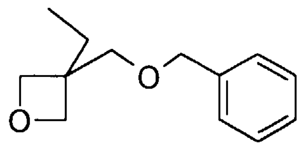
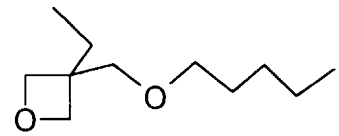

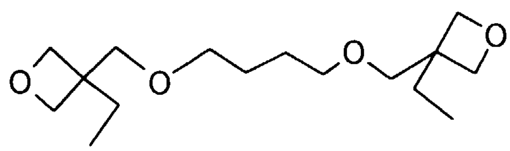

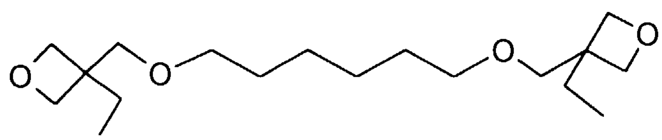

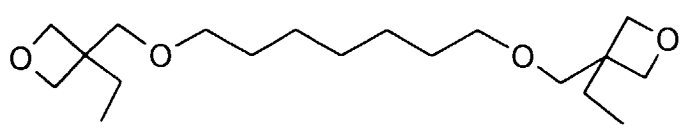

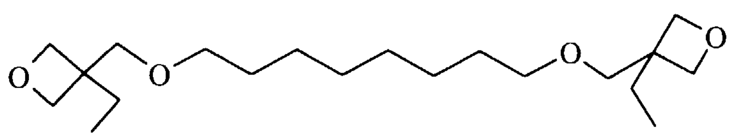

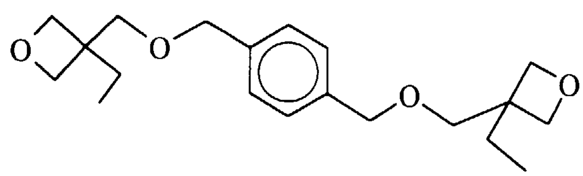

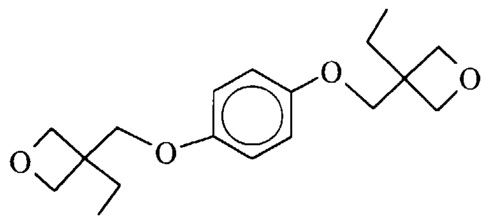

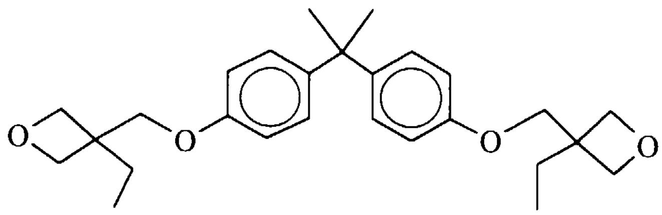

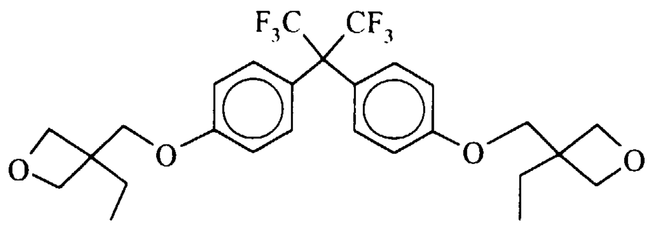

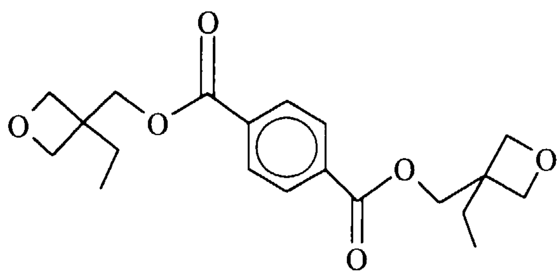

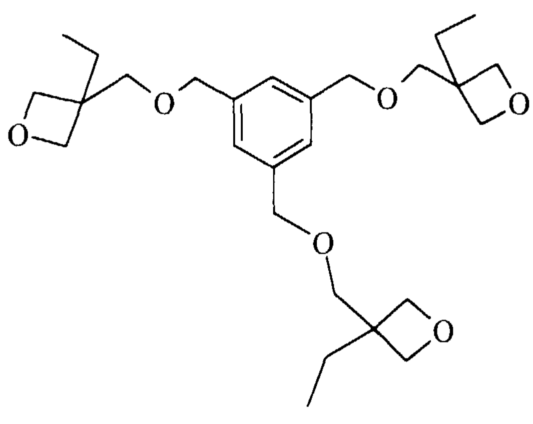

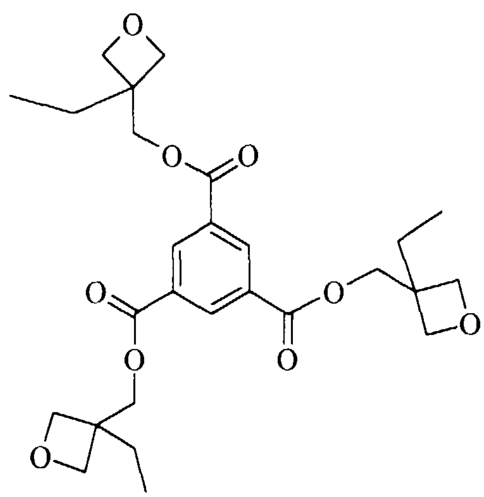

**2.** Matériau polymérisable selon la revendication 1, caractérisé en ce que la teneur en initiateur de polymérisation cationique est de 0,5-1,5% en poids et la teneur en initiateur de polymérisation radicalaire est de 0,3-1% en poids.

**3.** Matériau polymérisable selon la revendication 1 ou 2, caractérisé en ce que l'initiateur de polymérisation cationique est un sel de sulfonium pour la photopolymérisation.

**4.** Matériau polymérisable selon la revendication 1 ou 2, caractérisé en ce que l'initiateur de polymérisation cationique est un sel d'iodonium pour la photopolymérisation.

**5.** Matériau polymérisable selon l'une quelconque des revendications 1 à 4, caractérisé en ce qu'il contient 20-95% en poids du mélange et 5-80% en poids de charge organique finement divisée, de charge inorganique finement divisée ou d'un mélange de celles-ci.

**6.** Matériau polymérisable selon la revendication 5, caractérisé en ce que la charge organique finement divisée est constituée d'homo- ou de copolymères de méthacrylate d'alkyle.

**7.** Matériau polymérisable selon la revendication 5 ou 6, caractérisé en ce que la charge inorganique finement divisée est constituée de verre de silicate de lithium et d'aluminium, de verre de silicate de baryum et d'aluminium, de verre de silicate de strontium et d'aluminium, de verre de borosilicate de baryum et d'aluminium, de dioxyde de silicium ou d'un mélange de ceux-ci.

**8.** Utilisation du matériau polymérisable selon l'une quelconque des revendications 1 à 7 comme matériau dentaire.

**9.** Utilisation du matériau polymérisable selon l'une quelconque des revendications 1 à 7 comme ciment pour des os.

**10.** Utilisation du matériau polymérisable selon l'une quelconque des revendications 1 à 7 pour la préparation de revêtements sur de la céramique, du verre, du métal, du bois, du papier et de la matière plastique.